Europäisches Patentamt

European Patent Office

Office européen des brevets

(19)

(11) Publication number: **0 385 308**
**A1**

# EUROPEAN PATENT APPLICATION

(21) Application number: **90103566.7**

(22) Date of filing: **23.02.90**

(51) Int. Cl.5: **C12N 15/12, C07K 7/00,**
**C12P 21/02, C12N 1/18,**
**//(C12N1/18,C12R1:865)**

The applicant has filed a statement in accordance with Rule 28 (4) EPC (issue of a sample only to an expert). Accession number(s) of the deposit(s): FERM BP-2301.

(30) Priority: **28.02.89 JP 45495/89**
**29.03.89 JP 75090/89**

(43) Date of publication of application:
**05.09.90 Bulletin  90/36**

(84) Designated Contracting States:
**AT BE CH DE DK ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Onda, Haruo**
**201, 17-1 Namiki 3-chome**
**Tsukuba, Ibaraki 305(JP)**
Inventor: **Itoh, Yasuaki**
**202, 17-1 Namiki 3-chome**
**Tsukuba, Ibaraki 305(JP)**
Inventor: **Hayashi, Kyozo**
**828-1 Iwasaki**
**Gifu 502(JP)**

(74) Representative: **von Kreisler, Alek,**
**Dipl.-Chem. et al**
**Patentanwälte Von Kreisler-Selting-Werner**
**Deichmannhaus am Hauptbahnhof**
**D-5000 Köln 1(DE)**

(54) **Production of a peptide from human cancer cells.**

(57) Disclosed are (1) a DNA containing a DNA segment coding for a polypeptide having EAQ as the N-terminal sequence and composed of 60 amino acids, (2) a recombinant DNA constructed by introducing the DNA sequence in an expression vector for Escherichia coli, yeast or animal cells so as to express the foregoing polypeptide, (3) a transformant bearing the recombinant DNA and (4) a process for producing the foregoing polypeptide comprising cultivating the transformant, accumulating the polypeptide and collecting the same, whereby the polypeptide can be obtained in large amounts and is useful for the elucidation of the growth mechanism of cancer cells and the development of the therapeutic agents for carcinoma.

# PRODUCTION OF NOVEL POLYPEPTIDE

## BACKGROUND OF THE INVENTION

The present invention relates to a process for producing a polypeptide derived from a cancer cell, which comprises cloning cDNA coding for a polypeptide produced by the cancer cell, and producing the polypeptide by using the cloned cDNA by genetic engineering techniques.

Proteins or polypeptides secreted by certain kinds of cells are isolated and purified by various methods such as gel permeation chromatography, adsorption chromatography, electrophoresis and isoelectric point electrophoresis, using physiological activity to detect the presence of the polypeptide. However, if biologically active substances exist in small amounts, their isolation is accompanied by difficulty. For the purpose of solving this problem, it has been tried to produce the polypeptides in large amounts by genetic engineering techniques, and it has become possible to produce the polypeptides having reactivity equal to that of the polypeptides produced by the cells in immunoassay.

On the other hand, cancer cells have been known to secrete various polypeptides such as growth factors, and the relationship between these polypeptides and carcinomas has attracted much attention.

For example, human breast cancer cell MCF7 has been known to secrete the transforming growth factors (TGFs)$\alpha$, $\beta$ and $\gamma$ [Dickson et al., Cancer Res. 46, 1707-1713 (1986)], the insulin-like growth factor [Haff et al., Cancer Res. 46, 4613-4619 (1986)] and the platelet-derived growth factor [Brouzert et al., Pro. Natl. Acad. Sci. U.S.A. 84, 5763-5767 (1987)].

On the other hand, in order to examine the relationship between the MCF7 cells thus secreting various growth factors and an epidermal growth factor (hereinafter referred to as EGF) which is a kind of growth factor, one of the present inventors has established an enzyme immunoassay (hereinafter referred to as EIA) which can measure human EGF. EGF has been measured in cultures of MCF7 and human gastric cancer cells MKN-45 and KATO-III using the EIA. As a result, it has been discovered that a novel substance, different from EGF, is produced by these cells. It is conceivable that this substance has been detected because another antibody was present in the above human EGF assay system in addition to the antibody against EGF.

In order to obtain more information about the relationship between this novel polypeptide and carcinoma, this polypeptide has been purified and its structure has been clarified [Biochem. Biophys. Res. Comm. 155, 366 (1988)]. Based on these results, a patent application has been filed [Japanese Patent application No. 1-35111 (1989)]. This polypeptide has EAQ as the N-terminal sequence and is composed of 60 amino acids. This polypeptide is a novel polypeptide produced by human breast cancer cell MCF7 or human gastric cancer cell MKN-45 or KATO-III. Further, this polypeptide has a isoelectric point of 4.3, a molecular weight of 6,661 daltons and the following amino acid sequence:

EAQTETCTVAPRERQNCGFPGVTPSQCANKGCCFDDTVRGVPWCFYPNTIDVPPEEECEF

The novel polypeptide produced in accordance with the present invention will hereinafter sometimes be referred to as the "novel polypeptide".

An mRNA induced with estrogen in human breast cancer cells has been identified, and its complete nucleotide sequence has been elucidated by Chambon et al. The presence of a polypeptide (9140 daltons) composed of 84 amino acids has been predicted from this nucleotide sequence. A signal peptide sequence in the amino acid sequence was predicted, and it was further predicted that a polypeptide actually secreted may be one (6450 daltons) composed of 58 amino acids or one (6970 daltons) composed of 63 amino acids [Chambon et al., Nucleic Acids Research 12, No. 6, 2861-2878 (1984), and Chambon et al., DNA 4, 11-21 (1985)]. However, this is nothing more than an assumption, and such secreted polypeptides have neither been actually isolated nor purified to such a degree that their amino acid sequence can be determined.

Thus there has been a long felt need in this field for the isolation and purification of the polypeptides secreted by the breast cancer cells such as MCF7 and the like, particularly for the elucidation of any relationship between the polypeptide structures and carcinoma. However, it is difficult to isolate and purify polypeptides which are present in such small amounts. Further, it is difficult to extract stably a large amount of such polypeptides from natural sources. For these reasons, it is desirable to provide a process for producing this polypeptide in large amounts.

## SUMMARY OF THE INVENTION

It is therefore an object of the present invention to provide a process for producing a large amount of a novel polypeptide which human breast cancer cell MCF7 produces.

This invention provides a process for producing large amounts of this novel polypeptide by genetic recombination techniques which comprises synthesizing complementary DNA (cDNA) from mRNA obtained from human breast cancer cell MCF7 or the like, producing an expression vector containing DNA having a nucleotide sequence coding for this polypeptide based on the resulting cDNA, and introducing the resulting vector into Escherichia coli, yeast, animal cells or the like.

In accordance with the present invention, there are provided (1) a DNA sequence containing a DNA segment coding for a novel polypeptide having EAQ as the N-terminal sequence and composed of 60 amino acids, (2) a recombinant DNA constructed by introducing the DNA sequence into a vector for E. coli, yeast or animal cells so as to express the above polypeptide having EAQ as the N-terminal sequence and composed of 60 amino acids, (3) a transformant bearing the recombinant DNA, and (4) a process for producing the polypeptide which comprises cultivating the transformant, accumulating the polypeptide having EAQ as the N-terminal sequence and composed of 60 amino acids, and collecting the polypeptide thus obtained.

## BRIEF DESCRIPTION OF THE DRAWINGS

Fig. 1 is a scheme for the construction of a peptide expression vector according to the present invention, in which yeast is used as a host;

Figs. 2 and 3 are schemes for the construction of peptide expression vectors according to the present invention, in which animal cells are used as hosts;

Fig. 4 is an electrophoresis diagram with respect to identification of polypeptides according to the present invention which are produced by yeast and MCF7 cells; and

Figs. 5, 6 and 7 are graphs showing the course of purification of a polypeptide obtained by the present invention, showing the results of column chromatography, gel permeation chromatography and reverse-phase high performance liquid chromatography, respectively.

## DESCRIPTION OF THE PREFERRED EMBODIMENT

The expression vector having the DNA sequence containing the nucleotide sequence coding for the novel polypeptide produced in accordance with the present invention can be prepared, for example, by the following process:

(1) Messenger RNA (mRNA) is isolated from various cells producing the polypeptide in the present invention, such as MCF7 cells.

(2) Single stranded complementary DNA (cDNA) is synthesized by the mRNA, followed by synthesis of double stranded DNA.

(3) The complementary DNA is introduced into a plasmid or a phage.

(4) A host is transformed with the recombinant phage or plasmid thus obtained.

(5) After culturing of the transformant thus obtained, the plasmid or the phage containing the desired DNA is isolated from the transformant by an appropriate method such as hybridization with a DNA probe coding for a portion of the polypeptide or immunoassay using an anti-the peptide antibody which is specific for the novel polypeptide.

(6) The desired cloned DNA fragment is cut out from the recombinant DNA.

(7) The cloned DNA fragment or a portion thereof is ligated downstream from a promoter in the expression vector.

The mRNA coding for the polypeptide can be obtained from various cells which produce the polypeptide of the present invention, for example, human breast cancer cell MCF7 or human gastric cancer cell MKN-45 or KATO-III.

The methods for preparing the mRNA from these polypeptide-producing cells include the guanidine thiocyanate method [J. M. Chirgwin et al., Biochemistry 18, 5294 (1979)] and the like.

Using the mRNA thus obtained as a template, cDNA is synthesized by use of reverse transcriptase, for example, in accordance with the method of H. Okayama et al. [Molecular and Cellular Biology 2, 161 (1979); ibid. 3, 280 (1983)]. The cDNA thus obtained is introduced into a plasmid.

The plasmids into which the cDNA may be introduced include, for example, pBR322 [Gene 2, 95 (1977)], pBR325 [Gene 4, 121 (1978)], PUC12 [Gene 19, 259 (1982)] and PUC13 [Gene 19, 259 (1982)], each derived from E. coli, and pUB110 derived from Bacillus subtilis [Biochemical and Biophysical Research Communication 112, 678 (1983)]. However, any other plasmid can be used as long as it is replicable and growable in the host. The phage vectors into which the cDNA may be introduced include, for example, λgt11 [R. Young and R. Davis, Proc. Natl. Acad. Sci. U.S.A. 80, 1194 (1983)]. However, any other phage vector is appropriate, if growable in the host.

The methods for introducing the cDNA into the plasmid include, for example, the method described in T. Maniatis et al., Molecular Cloning, Cold Spring Laboratory, p.111 to 268 (1982). The methods for introducing the cDNA in the phage vector include, for example, the method of T. V. Hyunh et al. [DNA Cloning, A Practical Approach 1, 49 (1985)].

The plasmid thus obtained is introduced into appropriate cells such as E. coli and B. subtilis.

Examples of E. coli described above include E. coli K12DH1 [Proc. Natl. Acad. Sci. U.S.A. 60 160 (1968)], M103 [Nucleic Acids Research 9, 309 (1981)], JA221 [Journal of Molecular Biology 120, 517 (1978)], HB101 [Journal of Molecular Biology 41, 459 (1969)] and C600 [Genetics 39, 440 (1954)].

Examples of Bacillus described above include B. subtilis MI114 [Gene 24, 255 (1983)] and 207-21 [Journal of Biochemistry 95, 87 (1984)].

The methods for transforming the host with the plasmid include, for example, the calcium chloride method or the calcium chloride/rubidium chloride method both of which are described in T. Maniatis et al., Molecular Cloning, Cold Spring Harbor Laboratory, pp. 249 - 253(1982).

When a phage vector is used, for example, the phage vector can be transduced into rapidly growing E. coli, using the in vitro packaging method.

The polypeptide cDNA libraries containing the polypeptide cDNA can be obtained by the above-mentioned methods. Examples of the methods for detecting the cDNA from the MCF7 cDNA library include the colony hybridization or plaque hybridization method using oligonucleotide chemically synthesized on the basis of the amino acid sequences of the novel polypeptide as a probe [T. Maniatis, Molecular Cloning, Cold Spring Harbor Laboratory (1982)].

The polypeptide cDNA thus cloned is subcloned to a plasmid, for example, pBR322, PUC12, PUC13, PUC18, PUC19, pUC118 and pUC119 to obtain the polypeptide cDNA, if necessary.

The nucleotide sequence of the DNA thus obtained is determined by, for example, the Maxam-Gilbert method [A. M. Maxam and W Gilbert, Proc. Natl. Acad. Sci. U.S.A. 74, 560 (1977)] or the dideoxy method [J. Messing et al., Nucleic Acids Research 9, 309 (1981)], and the cDNA (formula I shown in Example 3 hereinafter described) coding for the polypeptide is compared to the known amino acid sequence of the novel polypeptide isolated from the cells disclosed above.

The DNA sequence coding for the polypeptide, cloned as described above, can be used as it is, or cut out by digestion with a restriction enzyme if desired, depending upon the intended use.

The region intended to be expressed is cut out from the cloned DNA and ligated downstream from a promoter in a vehicle (vector) suitable for expression, whereby the expression vector can be obtained.

The DNA sequence has ATG as a translation initiating codon at the 5′-terminus thereof and may have TAA, TGA or TAG as a translation terminating codon at the 3′-terminus thereof. There translation initiating codon and translation terminating codon may also be added by use of an appropriate synthetic DNA adaptor. Further, in order to express the DNA sequence, a promoter is ligated to the upstream thereof.

The vectors include the foregoing plasmids derived from E. coli, such as pBR322, PBR 325, PUC12 and PUC13, plasmids derived from yeast such as YIp, YEp, YRp and YCp, bacteriophage such as λphage, and animal viruses such as retroviruses and vaccinia viruses.

As the promoter used in the present invention, any promoter is appropriate as long as the promoter is suitable for expression corresponding to the host cell used for the gene expression.

When the host used for transformation is E. coli, it is preferable that a trp promoter, a lac promoter, a recA promoter, a λPL promoter, a lpp promoter and the like are used. When the host is B. subtilis, it is preferable that a SPO1 promoter, a SPO2 promoter, a penP promoter and the like are used. When the host is yeast, it is preferred that a PHO5 promoter, a PGK promoter, a GAP promoter, an ADH promoter and the like are used. Particularly, it is preferable that the host is E. coli and the promoter is the trp promoter or the λPL promoter.

When the host is an animal cell, a SV40-derived promoter, a retrovirus promoter, a metallothionein promoter, a heat shock promoter and the like can be used.

Also the use of an enhancer is effective for expression.

By using a vector containing the DNA sequence coding for the mature peptide of the polypeptide thus constructed, the transformant is prepared.

The host cells include, for example, E. coli, B. subtilis, yeast and animal cells.

Illustrative examples of E. coli and B. subtilis described above include the same strains as described above.

Examples of the yeast described above include Saccharomyces cerevisiae AH22, AH22R⁻, NA87-11A, and DKD-5D.

Examples of the animal cells include monkey cell COS-7, Vero, Chinese hamster cell (CHO), mouse L cell and human FL cell.

The transformation of E. coli described above is carried out, for example, according to the method described in Proc. Natl. Acad. Sci. U.S.A. 69, 2110 (1972) or Gene 17, 107 (1982).

The transformation of B. subtilis is conducted, for example, according to the method described in Molecular & General Genetics 168, 111 (1979).

The transformation of the yeast is carried out according to, for example, the method described in Proc. Natl. Acad. Sci. U.S.A. 75, 1929 (1978).

The transformation of the animal cells is carried out according to, for example, the method described in Virology 52, 456 (1973).

Thus, a transformant transformed with the expression vector containing the DNA sequence coding for the mature peptide of the polypeptide is obtained.

When the transformant wherein the host is Escherichia or Bacillus is cultivated, a liquid medium is suitable as a medium used for culture. Carbon sources, nitrogen sources, inorganic compounds and others necessary for growth of the transformant are contained therein. The carbon sources include, for example, glucose, dextrin, soluble starch and sucrose. The nitrogen sources include inorganic or organic materials such as ammonium salts, nitrates, corn steep liquor, peptone, casein, meat extracts, soybean meal and potato extract solution. The inorganic compounds include, for example, calcium chloride, sodium dihydrogenphosphate and magnesium chloride. Further, yeast, vitamins, growth promoting factors and so on may be added thereto.

The pH of the medium is preferably about 5 to 8.

As the medium used for cultivation of E. coli, there is preferred, for example, M9 medium containing glucose and Casamino Acids (Miller, Journal of Experiments in Molecular Genetics, 431-433, Cold Spring Harbor Laboratory, New York, 1972). In order to make the promoter act efficiently, a drug such as $3\beta$-indolylacrylic acid may be added thereto if necessary.

When the host is E. coli, the cultivation is usually carried out at about 15 to 43°C for about 3 to 24 hours, with aeration or agitation if necessary.

When the host is B. subtilis, the cultivation is usually carried out at about 30 to 40°C for about 6 to 24 hours, with aeration or agitation if necessary.

When a yeast transformant is cultivated, there is used, for example, Burkholder minimum medium [K. L. Bostian et al., Proc. Natl. Ace. Sci. U.S.A., 77, 4505 (1980)] as the medium. The pH of the medium is preferably adjusted to about 5 to 8. The cultivation is usually carried out at about 20 to 35°C for about 24 to 72 hours, with aeration or agitation if necessary.

When an animal cell transformant wherein the host is an animal cell is cultivated, examples of media include MEM medium containing about 5 to 20% fetal bovine serum [Science 122, 501 (1952)], DMEM medium [Virology 8, 396 (1959)], RPMI1640 medium [Journal of the American Medical Association 199, 519 (1967)] and 199 medium [Proceeding of the Society for the Biological Medicine 73, 1 (1950)]. The pH is preferably about 6 to 8. The cultivation is usually carried out at about 30 to 40°C for about 15 to 60 hours, with aeration or agitation if necessary.

The novel polypeptide can be isolated and purified from the culture described above, for example, by the following methods.

When the mature peptide is extracted from the cultivated cells, the cells are collected by a known method after the cultivation. Then, the collected cells are suspended in an appropriate buffer solution and disrupted by ultrasonic treatment, lysozyme and/or freeze-thawing. Thereafter, a crude extracted solution of the precursor protein of the polypeptide or the mature peptide thereof is obtained by centrifugation or filtration. The buffer solution may contain a protein denaturant such as urea or guanidine hydrochloride, or a surface-active agent such as Triton X-100.

When the precursor protein or the mature peptide is secreted into the culture medium, the supernatant is separated from the cells by a known method after the conclusion of cultivation, and then collected. The separation and purification of the precursor protein or the mature peptide contained in the culture supernatant or the extract thus obtained can be performed by an appropriate combination of known separating and purifying methods. The known separating and purifying methods include methods utilizing solubility such as salting out and solvent precipitation, methods mainly utilizing a difference in molecular weight such as dialysis, ultrafiltration, gel permeation chromatography and SDS-polyacrylamide gel electrophoresis, methods utilizing a difference in electric charge such as ion-exchange column chromatography, methods utilizing specific affinity such as affinity chromatography, methods utilizing a difference in hydrophobicity such as reverse-phase high performance liquid chromatography and methods utilizing a

5

difference in isoelectric point such as isoelectric focusing electrophoresis.

The activity of the precursor protein of the polypeptide or the mature peptide thereof thus formed can be measured by an enzyme immunoassay using an antibody specific for the precursor or mature peptide, or the like.

According to the present invention, the precursor protein of the polypeptide or the mature peptide thereof can be produced and purified in large amounts from the cells transfected or transformed with a vector containing the DNA sequence of the precursor or mature peptide.

The polypeptide produced in accordance with the present invention is produced and secreted from certain kinds of cancer cells, and anticipated to be related to carcinoma. For example, the measurement of the concentration or distribution of the polypeptide in cells or in tissues might make it possible to diagnose cancers or grasp a state of cancer development. Hence, this polypeptide can be useful for the elucidation of the growth mechanism of cancer and in turn the development of therapeutic agents for carcinoma. Further, this polypeptide has high homology with the known pancreatic spasmolytic enzyme (PSP) [Jorgensen et al., Regulatory Peptide 3, 207-219 (1982)] secreted from the pancreas, and can be assumed to have intestinal motility depression activity which the above enzyme has and further muscular contraction and relaxation activity.

When nucleotides, amino acids and so on are indicated by the abbreviations in this specification and drawings, the abbreviations adopted by IUPAC-IUB Commission on Biochemical Nomenclature or commonly used in the art are employed. For example, the following abbreviations are used. When optical isomers are capable of existing with respect to the amino acid, an L-form is represented unless otherwise specified.

DNA : Deoxyribonucleic acid
cDNA : Complementary deoxyribonucleic acid
A : Adenine
T : Thymine
G : Guanine
C : Cytosine
RNA : Ribonucleic acid
mRNA : Messenger ribonucleic acid
dATP : Deoxyadenosine triphosphate
dTTP : Deoxythymidine triphosphate
dGTP : Deoxyguanosine triphosphate
dCTP : Deoxycytidine triphosphate
ATP : Adenosine triphosphate
EDTA : Ethylenediaminetetraacetic acid
SDS : Sodium dodecyl sulfate
Gly or G : Glycine
Ala or A : Alanine
Val or V : Valine
Leu or L : Leucine
Ile or I : Isoleucine
Ser or S : Serine
Thr or T : Threonine
Cys or C : Cysteine
Met or M : Methionine
Glu or E : Glutamic acid
Asp or D : Aspartic acid
Lys or K : Lysine
Arg or R : Arginine
His or H : Histidine
Phe or F : Phenylalanine
Tyr or Y : Tyrosine
Trp or W : Tryptophan
Pro of P : Proline
Asn or N : Asparagine
Gln or Q : Glutamine

The present invention will hereinafter be described in detail with the following Examples. It is understood of course that these Examples are not intended to limit the scope of the invention.

Transformant S. cerevisiae AH22R⁻/pGLD906-10 obtained in Example 4 described below was deposited in Fermentation Research Institute, Agency of Industrial Science and Technology, Ministry of International Trade and Industry, Japan (FRI) under the accession number FERM BP-2301 on February 21, 1989. This microorganism was also deposited in the Institute for Fermentation, Osaka, Japan (IFO) under the accession number IFO 10465 on February 28, 1989.

Example 1

Preparation of cDNA Library from MCF7 Cells

MCF7 cells were monolayer cultivated in Eagle's minimal essential medium containing 10% fetal bovine serum. RNA was extracted from $10^8$ cells thus cultivated by the guanidine-hot phenol method (T. Maniatis et al., Molecular Cloning-A Laboratory Manual p.194-195, 1982). The RNA thus extracted was purified by oligo(dT) cellulose column chromatography to provide poly(A) RNA (ibid. p.197-198). Using the poly(A) RNA as a template, a cDNA library was synthesized by the method of H. Okayama.

Example 2

Preparation of DNA Probe Coding for Part of Polypeptide

From the DNA sequence coding for the amino acid sequence composed of the 9th Val to the 15th Gln of the novel polypeptide, Val-Ala-Pro-Arg-Glu-Arg-Gln, the codon having the highest usage frequency was selected according to the method of R. Lathe [J. Mol. Biol. 183, 1-12 (1985)], and a DNA probe having the following sequence was synthesized:
⁵ GTG GCC CCC CGT GAA AGA CAG³
The 5′-terminus of this DNA probe was phosphorylated with $^{32}$p by using T4 polynucleotide kinase. The DNA probe thus phosphorylated was used for screening the cDNA library.

Example 3

Isolation of Polypeptide cDNA and Determination of Nucleotide Sequence Thereof

E. coli C600hfl was infected with the foregoing cDNA library and plated to make colonies appear. The colonies were transferred to a nylon film and hybridized with the DNA probe labeled with $^{32}$p in Example 2. The hybridization was carried out at 42°C. Each of several clones positive to hybridization was isolated. Then, the nucleotide sequence of a cDNA portion of No. 52 which was one of the clones described above was determined. The cDNA portion included in this plasmid was 1.8 Kbp. The nucleotide sequence of the cDNA portion was determined by the method of Sanger [Proc. Natl. Acad. Sci. U.S.A. 74, 5463-5467 (1977)-]. This nucleotide sequence and the amino acid sequence of the precursor protein presumed therefrom are shown in formula 1. The region surrounded by □ shows the polypeptide obtained in the present invention.

Formula I

```
         A TCC CTG ACT CGG GGT CGC CTT     22

                                 Met Ala    2
    23  TGG AGC AGA GAG GAG GCA ATG GCC    46

     3  Thr Met Glu Asn Lys Val Ile Cys    10
    47  ACC ATG GAG AAC AAG GTG ATC TGC    70

    11  Ala Leu Val Leu Val Ser Met Leu    18
    71  GCC CTG GTC CTG GTG TCC ATG CTG    94

    19  Ala Leu Gly Thr Leu Ala Glu Ala    26
    95  GCC CTC GGC ACC CTG GCC GAG GCC   118

    27  Gln Thr Glu Thr Cys Thr Val Ala    34
   119  CAG ACA GAG ACG TGT ACA GTG GCC   142

    35  Pro Arg Glu Arg Gln Asn Cys Gly    42
   143  CCC CGT GAA AGA CAG AAT TGT GGT   166

    43  Phe Pro Gly Val Thr Pro Ser Gln    50
   167  TTT CCT GGT GTC ACG CCC TCC CAG   190

    51  Cys Ala Asn Lys Gly Cys Cys Phe    58
   191  TGT GCA AAT AAG GGC TGC TGT TTC   214

    59  Asp Asp Thr Val Arg Gly Val Pro    66
   215  GAC GAC ACC GTT CGT GGG GTC CCC   238

    67  Trp Cys Phe Tyr Pro Asn Thr Ile    74
   239  TGG TGC TTC TAT CCT AAT ACC ATC   262

    75  Asp Val Pro Pro Glu Glu Glu Cys    82
   263  GAC GTC CCT CCA GAA GAG GAG TGT   286

    83  Glu Phe ***                        84
   287  GAA TTT TAG ACA CTT CTG CAG GGA   310

   311  TCT GCC TGC ATC CTG ACG GGG TGC   334
```

```
335   CGT CCC CAG CAC GGT GAT TAG TCC   358

359   CAG AGC TCG GCT GCC ACC TCC ACC   382

383   GGA CAC CTC AGA CAC GCT TCT GCA   406

407   GCT GTG CCT CGG CTC ACA ACA CAG   430

431   ATT GAC TGC TCT GAC TTT GAC TAC   454

455   TCA AAA TTG GCC TAA AAA TTA AAA   478

479   GAG ATC GAT ATT                   490
```

Example 4

Construction of Polypeptide Expression Vector to be Used in System Wherein Host is Yeast and Transduction into Yeast

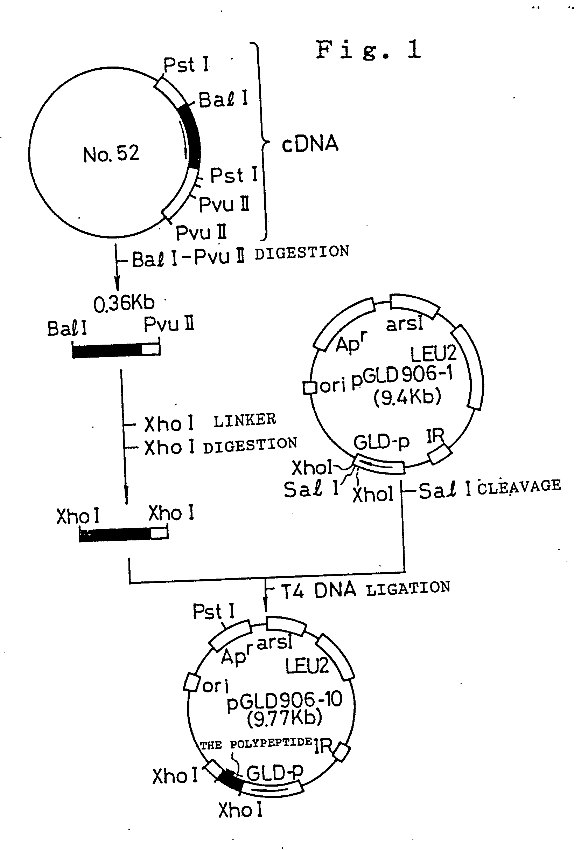

The plasmid No. 52 (20 μg) described in Example 3 was digested with 40 units of each of restriction enzymes BalI and PvuII (both Takara Syuzo Inc.). Thereafter, a DNA fragment of 0.36 Kbp was isolated by 1.5% agarose gel electrophoresis. To this DNA fragment (2 μg), 0.1 μg of XhoI linker d(CCTCGAGG) (Takara Syuzo Inc.) was added and ligated with 200 units of T4 DNA ligase (Takara Syuzo Inc.) in 50 μl of a reaction solution (66 mM Tris-HCl, pH 7.6/6.6 mM MgCl₂/10 mM dithiothreitol/0.1 mM ATP) at 14°C for 16 hours. Then, 30 units of restriction enzyme XhoI (Nippon Gene Inc.) was added thereto and reacted at 37°C for 2 hours to trim both ends of the DNA fragment. 0.5 μg of this DNA fragment was linked with 0.1 μg of a 9.4 Kbp DNA fragment obtained by digesting expression vector pGLD906-1 for yeast (Japanese Patent Unexamined Publication No. 61- 43991/1986) with restriction enzyme SalI, by use of 200 units of T4 DNA ligase in the reaction solution described above (50 μl) to transform E. coli DHI (Molecular Cloning, Cold Spring Harbor Laboratory, 1982). Plasmid pGLD906-10 was isolated from the ampicillin-resistant transformant thus obtained (Fig. 1).

Using 3 μg of plasmid pGLD906-10, S. cerevisiae AH22R⁻ [Miyanohara et al., Proc. Natl. Acad. Sci. U.S.A. 80, 1 (1983)] was transformed by the protoplast method [Hinnen et al., Proc. Natl. Acad. Sci. U.S.A. 75, 1927 (1978)]. As a result, transformant AH22R-/pGLD906-10 viable on a leucine-free medium was isolated.

Example 5

Expression of Polypeptide in System Wherein Host is Yeast

Production of Polypeptide

From many strains of transformant S. cerevisiae AH22R⁻/pGLD906-10 obtained in Example 4, several strains were selected, and the polypeptide productivity thereof was investigated by the following method.

The medium of Kitano et al. [BIO/TECHNOLOGY 5, 281 (1987)] was dispensed in 5 ml portions into test tubes, and the transformant was inoculated thereto. Then, the culture was shaken at 30°C for 3 days. Further, 1 ml of each culture was transferred into each of test tubes into which the same medium had been dispensed in 10 ml portions, and the culture was shaken at 30°C for 1 day. Subsequently, 3 ml of each culture thus obtained was transferred into each of 200 ml flasks containing the same medium as described above in 30 ml portions and cultivated at 30°C for 2 days with shaking.

The culture thus obtained were fractionated by centrifugation at 3,000 rpm for 10 minutes to separate into supernatants and cells. The cells were disrupted by the method of Rose et al. [Proc. Natl. Acad. Sci. U.S.A. 78, 2460 (1981)] to obtain extract. Namely, the cells collected from 10 ml of each culture solution were once washed with SM buffer (85 mM NaCl, 1 mM MgSO₄. 20 mM Tris-HCl, pH 7.4) and then frozen at -80°C. To the frozen cells, 1 ml of a buffer for disruption (100 mM Tris-HCl, pH 8.0, 20% Glycerol, 1 mM PMSF, 1 mM DTT) and 2 g of glass beads were added, and the cells were disrupted under vigorous agitation by a vortex mixer. The resultant products were centrifuged to obtain supernatants as cell extracts.

The presence of the novel polypeptide was detected by EIA as shown below.

Plate Sensitization

200 μg/ml of an IgG fraction (purified from rabbit serum) containing anti-hEGF antibody was added to 50 mM of Tris-HCl (pH 8.0), and the mixture was poured to a microplate having 96 wells (Sumitomo Bakelite Co.) in an amount of 100 μl/well. After the reaction was conducted at 37°C for 3 hours, the solution was discarded. Buffer A was further added thereto and blocking was carried out. Then, the resultant product was further allowed to stand at 4°C overnight. The composition of the buffer A is as follows:

0.1 M phosphate buffer (pH 7.0)
0.1% bovine serum albumin (BSA)
0.3 M NaCl
0.1% NaN₃
1 mM MgCl₂

Using this assay system, an assay was performed in the following manner.

Assay

A plate was washed once with 200 μl/well of buffer A just prior to the use thereof. Then, 100 μl/well of buffer A and 100 μl/well of a sample were added to the plate in duplicate, and reacted with each other at 4°C overnight, followed by washing with four 200 μl/well portions of a washing buffer. 100 μl/well of Fab'- HRP conjugate (which was prepared by diluting 1000 times a liquid concentrate with the washing buffer) was added thereto, and reacted at 37°C for 4 hours. Then, the reaction product was washed with four 200 μl/well portions of the washing buffer, and 100 μl/well of 0.6% HPPA[3-(p-hydroxyphenyl)propionic acid] solution in 0.1 M phosphate buffer (pH 7.0) and 100 μl/well of 0.015% H₂O₂ were added thereto. Then, the reaction was conducted at 37°C for more than 1 hour, and terminated with 50 μl/well of 0.1 M glycine-NaOH (pH 10.3). The resultant product was transferred to a DYNATECH™ MICROFLUOR (for fluorescenct enhancer), and the fluorescence was measured with an immunoreader (365 nm excitation, 415 nm fluorescence measurement). The following were used as controls.

Negative: H₂O (0)
Positive: 1.0 μg/ml of kinin in 0.1 N sulfuric acid (100)

The data of Table 1 below shows that transformants were obtained which produced the novel polypeptide both inside and outside the cells.

Table 1

| | Inside cells | Outside cells |
|---|---|---|
| 1. Yeast | Total amount in 200 μl ng/tube | Total amount in medium (1 ml) ng |
| B (Disruption solution alone) | 0 | |
| 1 AH22R⁻ No.1 | 54.1 | 202 |
| 2 AH22R⁻ No.3 | 52.0 | |
| 3 AH22R⁻ No.5 | 54.8 | |
| 4 AH22R⁻ No.7 | 66.1 | 230 |
| 5 AH22R⁻ No.9 | 59.8 | 227 |
| 6 AH22R⁻ C | 0 | 0 |
| C: Control having no vector | | |

## Example 6

Expression of Polypeptide in Animal Cells

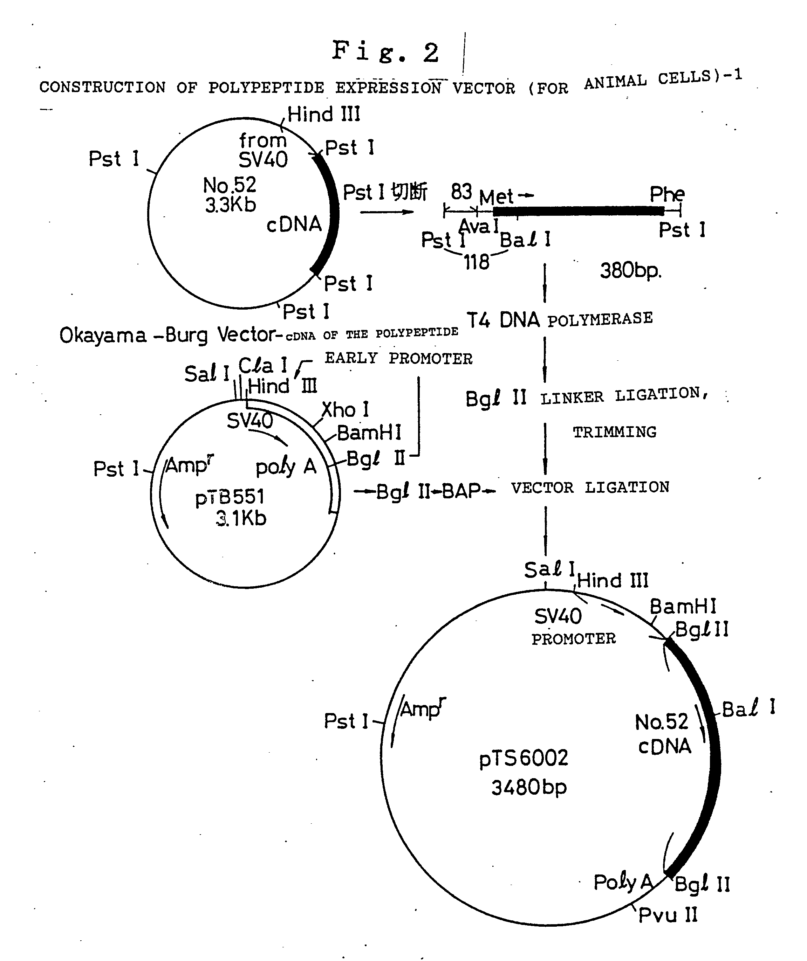

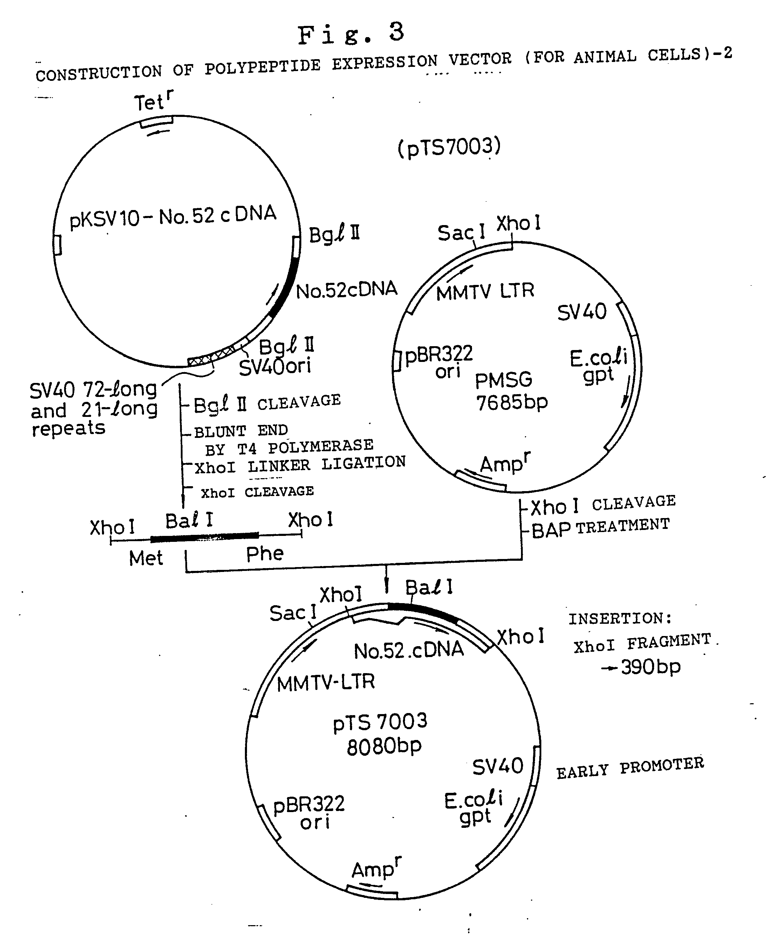

For the purpose of expressing the polypeptide in animal cells such as COS7 cell and CHO cell, a plasmid was constructed in a manner as shown in Figs. 2 and 3.

Namely, the plasmid No. 52 was purified, cleaved with PstI and then treated with T4 polymerase, followed by linkage of BglII linker. Thereafter, the resultant product was introduced into the BglII site of pTB551 to obtain pTS6002.

The plasmid thus obtained was introduced into animal cell COS7, and the production of the polypeptide was examined by EIA. As a result, it was confirmed that the polypeptide was secreted in the culture outside the cells as shown in Table 2 below. Further, its existence inside the cell was also confirmed.

Table 2

| Specimen | Outside cells (pg/ml) | Inside cells (pg/ml) |
|---|---|---|
| 1 | 33.5 | 10.8 |
| 2 | 38.5 | 37.5 |
| 3 | 44.0 | 30.0 |
| 4 | 36.5 | 51.0 |

Using CHO cells (Chinese hamster ovary cells) transformed with plasmid pTS7003 synthesized in a manner shown in Fig. 3, cell strains producing the polypeptide were obtained in amounts of 1020, 3750, 1700, 2150 and 1070 pg/ml, respectively.

## Example 7

Identification of Polypeptide Produced by Yeast and MCF7 Cell

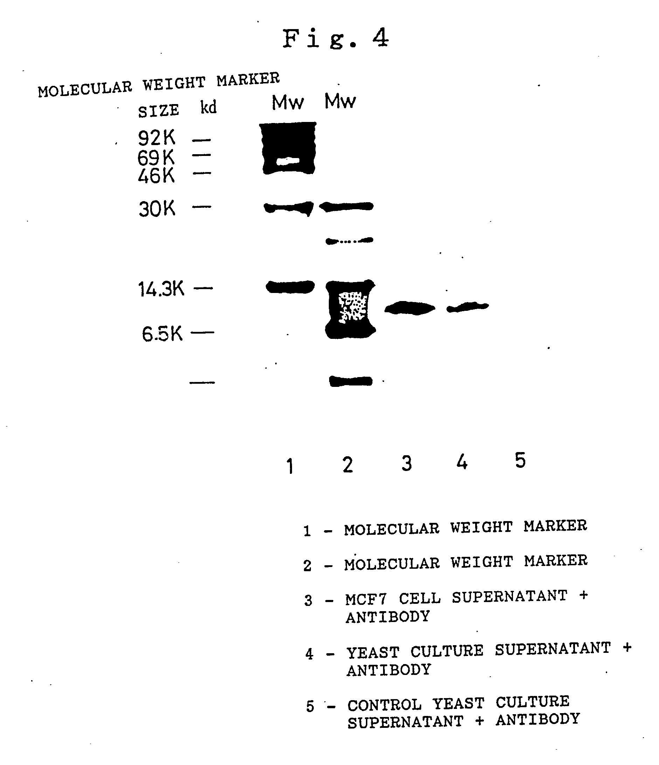

The yeast transformant AH22R⁻/pGLD906-10 was cultivated in the presence of $^{35}$S-cysteine, and the antibody used in the EIA for the measurement of the polypeptide was added to its supernatant. Then, the mixture was allowed to stand at 37°C for 1 hour and further at 4°C overnight. Thereafter, Protein A Sepharose was added thereto to produce an antigen-antibody complex. The complex was collected, followed by 20% polyacrylamide gel electrophoresis. After drying, the determination was carried out by autoradiography. Consequently, the polypeptide was identified at the position corresponding to a molecular weight of about 8,000 daltons as a single band. After $^{35}$S-cysteine was added to a culture, the MCF7 cells were similarly cultivated. Then, its supernatant was analyzed by polyacrylamide gel electrophoresis in the same manner as described above (Fig. 4). The polypeptide thus expressed can be purified in large amounts and used for the studies of physiological activity.

## Example 8

Purification of Polypeptide Produced by Yeast and Determination of N-Terminal Amino Acid Sequence

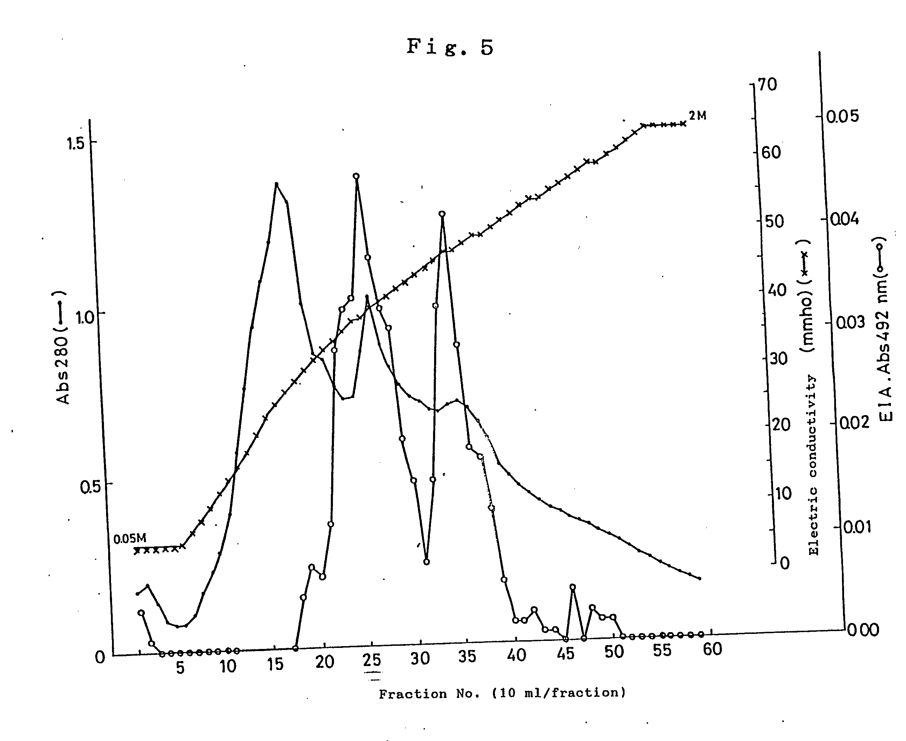

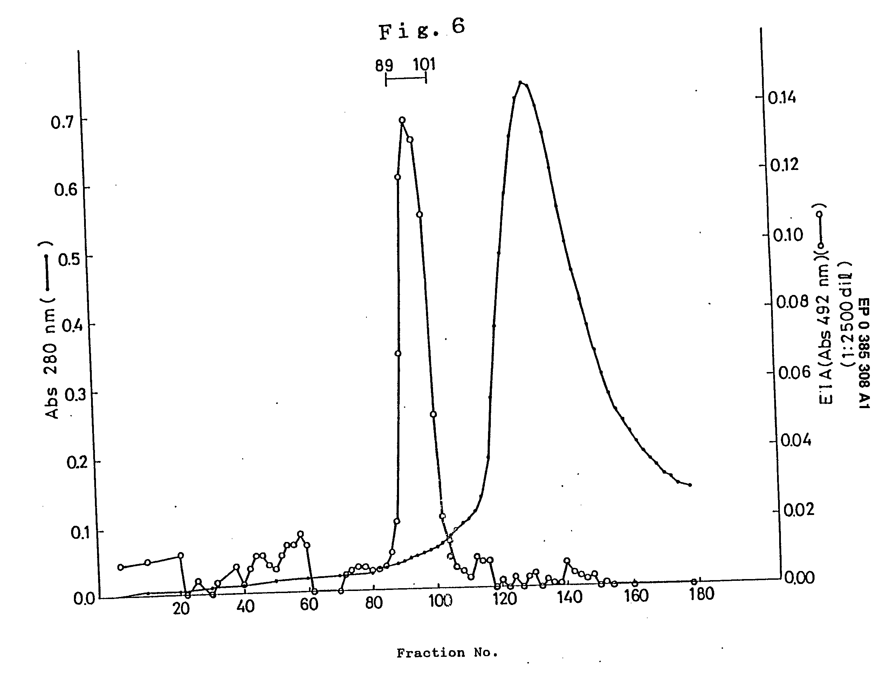

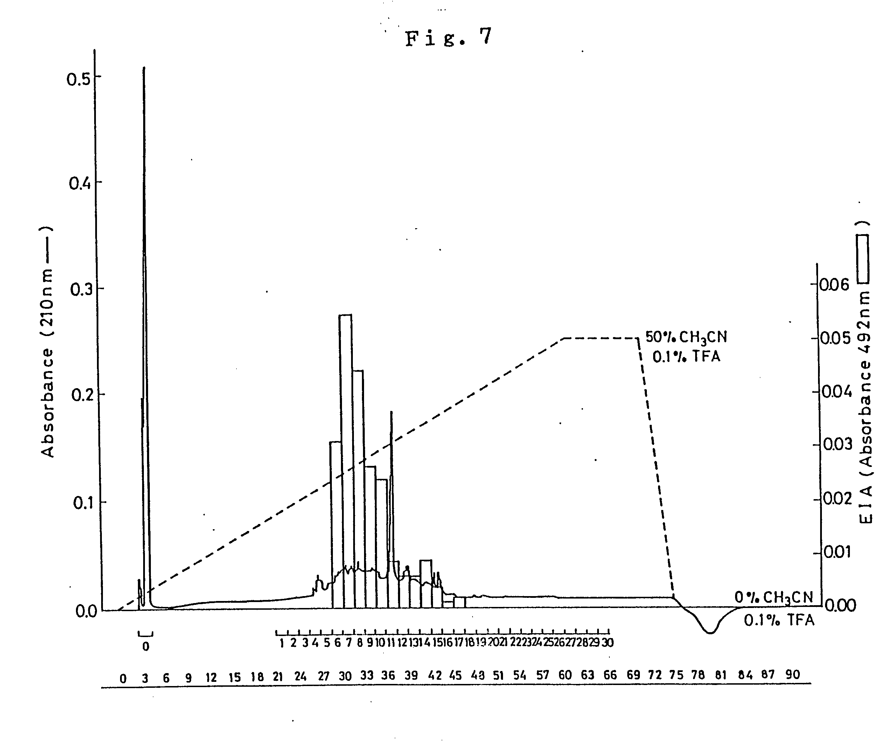

1 liter of the yeast (AH22R⁻/pGLD906-10) culture supernatant obtained in Example 7 described above was dialyzed with Spectrapor 3 (registered trade mark, Spectrum Medical Ind.) against 1% acetic acid, and dissolved in 25 ml of 0.05 M ammonium acetate (pH 5.5) after freeze-drying. A precipitate was removed by centrifugation at 3,000 rpm at 4°C for 10 minutes. The resultant solution was subjected to DEAE-Sephadex A-25 column chromatography (Fig 5), gel permeation chromatography using a Sephadex G-50 superfine column (Fig. 6) and reverse-phase high performance liquid chromatography (HPLC) (Fig. 7). Then, positive EIA fraction No. 7 was used for the determination of the amino acid sequence.

In DEAE-Sephadex A-25 column chromatography, a column 1.6 cm in diameter, 43 cm in length and 100 ml in bed volume was used, and elution was carried out at a flow late of 0.8 ml/minute by a linear gradient of ammonium acetate (pH 5.5) 0.05 to 2 M, followed by fractionation at 10 ml/fraction by use of a Pharmacia FPLC system. The absorbance, electric conductivity and EIA (enzyme immunoactivity) of these fractions at A280 nm were measured. The results are shown in Fig. 5. Portions of fraction Nos. 21 to 30 corresponding to the polypeptide were lyophilized and then dissolved in 10 ml of 1% acetic acid, followed by transferring to gel permeation chromatography.

In the gel permeation chromatography, a Sephadex G-50 superfine column 2.5 cm in diameter, 108 cm in length and 500 ml in bed volume was used, and elution was carried out at a flow late of 0.2 ml/minute by 1% acetic acid, followed by fractionation at a ratio of 4 ml/fraction. The absorbance, electric conductivity and EIA at A280 nm were measured. The results are shown in Fig. 6. Portions of fraction Nos. 89 to 101 were lyophilized and then dissolved in 100 $\mu$l of 10% TFA, followed by transferring to reverse-phase high performance liquid chromatography.

In reverse-phase high performance liquid chromatography, a column (Wakapak Wacosil 10C$_{18}$, registered trade mark, Wako Pure Chemical Ind.) 4.6 mm in diameter and 250 mm in length was used, and a linear gradient of 0.1% TFA containing 0 to 50% CH$_3$CN was made to flow therein at a flow rate of 1.0 ml/minute, followed by fractionation at a ratio of 1.5 ml/fraction. Then, the measurement of EIA was carried out. The results are shown in Fig. 7. The sample (fraction No. 7) was lyophilized and then dissolved in 20% acetonitrile, followed by Edman degradation. As a result of analysis according to a conventional method, the N-terminal sequence showed EAQTETCTVAP when each amino acid was represented by one-literal notation. This sequence is identical with the 25th to 35th residues of the novel polypeptide secreted by human breast cancer cell MCF7 and described above. Thus, it became clear that the polypeptide obtained in accordance with the present invention was the same as the polypeptide secreted by MCF7 cells (Japanese Patent Application No. 1-35111/1989).

## Claims

1. A DNA containing a DNA segment coding for a polypeptide having EAQ as its N-terminal sequence and composed of 60 amino acids.

2. A DNA according to claim 1, wherein said polypeptide has an isoelectric point of about 4.3.

3. A DNA according to claim 2 or 3, wherein said polypeptide has a molecular weight of about 6,661.

4. A DNA according to claim 1, wherein said polypeptide has the following amino acid sequence:
EAQTETCTVAPRERQNCGFPGVTPSQCANKGCCFDDTVRGVPWCFYPNTIDVPPEEECEF

5. A DNA according to claim 1, wherein said DNA is a cDNA sequence synthesized from a messenger RNA (mRNA) derived from human breast cancer cell MCF7 or human gastric cancer cell MKN-45 or KATO-III.

6. A recombinant DNA constructed by introducing the DNA as claimed in claim 1, 2, 3, 4 or 5 into a vector capable of expression in Escherichia coli, yeast or animal cells, which produces a polypeptide having EAQ as the N- terminal sequence and composed of 60 amino acids.

7. A recombinant DNA according to claim 6, further comprising a signal sequence which allows the polypeptide to be secreted in a culture medium.

8. A transformant bearing the DNA as claimed in claim 6 or 7.

9. A transformant according to claim 8, wherein the transformant host is Escherichia coli, yeast or animal cells except for human breast cancer cell MCF7, human gastric cancer cells MKN-45 and KATO-III.

10. Saccharomyces cerevisiae AH22R$^-$/pGLD 906-10 (FERM BP-2301) according to claim 9.

11. A process for producing a polypeptide having EAQ as the N-terminal sequence and composed of 60 amino acids, which comprises cultivating the transformant according to claim 8, 9 or 10, accumulating the polypeptide in a culture medium, and then collecting the polypeptide.

12. A process according to claim 11, wherein said polypeptide is modified by site-directed mutagenesis.

EP 0 385 308 A1

Fig. 1

EP 0 385 308 A1

# Fig. 2

CONSTRUCTION OF POLYPEPTIDE EXPRESSION VECTOR (FOR ANIMAL CELLS)-1

Okayama –Burg Vector-cDNA OF THE POLYPEPTIDE

EARLY PROMOTER

T4 DNA POLYMERASE

Bgl II LINKER LIGATION,

TRIMMING

—Bgl II-BAP— VECTOR LIGATION

# Fig. 3

CONSTRUCTION OF POLYPEPTIDE EXPRESSION VECTOR (FOR ANIMAL CELLS)-2

# Fig. 4

MOLECULAR WEIGHT MARKER

SIZE  kd    Mw   Mw

92K —
69K —
46K —

30K —

14.3K —

6.5K —

—

1    2    3    4    5

1 - MOLECULAR WEIGHT MARKER

2 - MOLECULAR WEIGHT MARKER

3 - MCF7 CELL SUPERNATANT +
    ANTIBODY

4 - YEAST CULTURE SUPERNATANT +
    ANTIBODY

5 - CONTROL YEAST CULTURE
    SUPERNATANT + ANTIBODY

Fig. 5

EP 0 385 308 A1

Fig. 6

EP 0 385 308 A1

Fig. 7

EP 0 385 308 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | DNA, vol. 4, no. 1, 1985, pages 11-21, New York, US; J.-F. PRUD'HOMME et al.: "Cloning of a gene expressed in human breast cancer and regulated by Estrogen in MCF-7 cells" * Whole document * | 1-12 | C 12 N 15/12<br>C 07 K 7/00<br>C 12 P 21/02<br>C 12 N 1/18 //<br>(C 12 N 1/18<br>C 12 R 1:865) |
| D,A | BIOCHEMICAL AND BIOPHYSICAL RESEARCH COMMUNICATIONS, vol. 155, no. 1, 30th August 1988, pages 366-372, Academic Press, Inc., Duluth, Mn, US; K. MORI et al.: "Identification of a polypeptide secreted by human breast cancer cells (MCF-7) as the human estrogen-responsive gene (pS2) product" * Whole document * | | |

TECHNICAL FIELDS SEARCHED (Int. Cl.5)

C 12 N
C 12 P

The present search report has been drawn up for all claims

| Place of search | Date of completion of the search | Examiner |
|---|---|---|
| THE HAGUE | 23-04-1990 | VAN PUTTEN A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)